# EUROPEAN PATENT APPLICATION

(11) **EP 4 578 300 A1**
(43) Date of publication of application: **02.07.2025**
(21) Application number: 24221312.2
(22) Date of filing: 18.12.2024
(51) Int. Cl.: A23L 33/15, A61K 31/198, A61K 31/205, A61K 31/335, A61K 31/355, A61K 33/30

(54) **DIETARY SUPPLEMENT BASED ON NON-ALCOHOLIC BEER SUPPORTING MALE FERTILITY**

(30) Priority: 18.12.2023 PL 44713323
(71) Applicant: Fertilbrew Sp. z o.o., 55-011 Siechnice (PL)
(72) Inventor: Wrobel, Michal, 55-011 Siechnice (PL)
(74) Representative: Czarnik, Maciej

(57) **Abstract**

The subject of the invention is a dietary supplement based on non-alcoholic beer supporting male fertility, characterized in that it comprising L-carnitine in an amount of 0.16-0.24% w/w, L-arginine in an amount of 0.02-0.06% w/w, zinc in an amount of 0.002-0.003% w/w, selenium in an amount of 0.00002-0.00004% w/w, vitamin C in an amount of 0.02-0.2% w/w, vitamin E in an amount of 0.01-0.03% w/w, vitamin B6 in an amount of 0.00028-0.002% w/w, vitamin B12 in an amount of 0.0000005-0.000006% w/w, methylfolate in an amount of 0.00008-0.00012% w/w, myo-inositol in an amount of 0.0001-0.0002% w/w, resveratrol in an amount of 0.01-0.04% w/w, choline in an amount of 0.01-0.04% w/w, lycopene in an amount of 0.0002-0.002% w/w, coenzyme Q10 in an amount of 0.001 -0.004% w/w.

## Description

The subject of the invention is a dietary supplement based on non-alcoholic beer that supports male fertility by providing essential micronutrients and vitamins, intended for use in the food and pharmaceutical industries.

Infertility has become a civilization disease in recent years. According to the latest WHO data, it affects approximately 186 million people worldwide, accounting for 15% of couples of reproductive age. It is well known that one of the significant factors negatively affecting semen quality is an improper diet, leading to a decreased supply of vitamins and micronutrients essential for the proper functioning of the body.

Therefore for men of reproductive age trying to conceive, proper nutrition plays a crucial role by ensuring an adequate supply of nutrients. In addition to providing all necessary dietary components for restoring or maintaining the body's homeostasis, a diet should also consider proper hydration and appropriately selected supplementation.

Idiopathic male infertility is associated with impaired semen parameters without an identifiable cause in standard medical examinations. The main cause of this condition, apart from congenital genetic changes, is an excess of free radicals (oxidative stress), which damage sperm genetic material. The body has mechanisms to reduce free radicals, but the proper functioning of these mechanisms requires an adequate supply of micronutrients and vitamins. Many environmental and lifestyle factors, such as pollution, poor diet, excessive alcohol consumption, and smoking, contribute to free radical production while simultaneously disrupting the supply and absorption of essential nutrients needed for optimal sperm production. Reduced intake and impaired absorption of certain elements and vitamins negatively impact spermatogenesis, leading to various mechanisms of fertility disorders.

Studies have shown that certain micronutrients and vitamins, such as myo-inositol, L-arginine, coenzyme Q10, L-carnitine, selenium, zinc, and resveratrol, significantly improve sperm production and quality by reducing oxidative stress.

L-carnitine enhances sperm vitality and motility, serves as an energy source, and protects DNA and cell membranes from free radical damage [1, 2, 3].

Coenzyme Q10 is closely related to the mitochondrial respiratory chain and plays a role in gene expression modulation, cellular communication, transport, and metabolism. Studies show that men with reduced fertility have lower levels of coenzyme Q10, and for patients with infertility due to oligoasthenoteratozoospermia, the administration of coenzyme Q10 resulted in improved semen quality (spermatozoa quantity, viability and motility) [1, 2, 3, 4, 5, 6].

L-arginineis an important amino acid that plays a variety of roles in animal metabolism. In relation to fertility disorders, a positive effect of oral administration of L-arginine on sperm count and motility has been proven [7, 8].

Myo-inositol is a carbohydrate that is a precursor of phosphatidylinositol polyphosphates, which are key components in intracellular signal transduction. In vitro and in vivo supplementation experiments confirm the role of myoinositol in improving sperm motility and viability [9, 10].

Selenium is an antioxidant and thus has a protective effect on semen during sperm maturation. Selenium supplementation has also been shown to reduce the risk of developing prostate cancer by 10-49% [11, 12, 13, 14].

The concentration of zinc correlates with semen quality-it is significantly lower in infertile patients or those with reduced fertility. This effect is attributed to the antioxidant properties of zinc, which help maintain developing sperm in optimal condition. Additionally, zinc has been proven to play a crucial role in ensuring the proper function of the acrosome, which is necessary for the sperm head to penetrate the egg cell. It has also been observed that smoking men have lower serum zinc levels, which may be one of the mechanisms by which smoking negatively affects male fertility. Zinc, when administered in physiological doses, is essential for maintaining proper testosterone production [15, 16, 17, 18, 19, 20].

Vitamin E is also an antioxidant, and this mechanism is considered particularly important in its protective effect on sperm [11, 20, 21].

Vitamin A is responsible for the proper function of the epithelium of the epididymis, seminal vesicles, and prostate. Additionally, its role in the early stages of spermatogonial division has been demonstrated [22, 23].

Lycopene, (carotenoids found in vegetables, primarily in tomatoes and their derivatives), when supplemented at doses of 4-8 mg for 3-12 months, improve sperm motility by over 50%. This effect is mainly due to the reduction of oxidative stress. The same mechanisms are responsible for the protective effect of lycopenes in preventing the development of prostate cancer, reducing the risk by 30-40% [24, 25, 26].

Vitamin B6 (pyridoxine) is one of the factors that protect against toxic damage to sperm. Lower concentrations of vitamin B6 have been demonstrated in a group of men with asthenozoospermia compared to those with normal fertility [27].

Methylfolate and other derivatives of folic acid play a significant role in maintaining male fertility, although their exact mechanism is not fully understood-most likely, it involves a protective role in maintaining DNA integrity. Reduced concentrations of folates have been demonstrated in patients with fertility disorders [28, 29, 30].

Choline is, among other things, a component of cell membranes and has been shown to have particularly high concentrations in epididymal cells. Proper membrane structure is essential for the normal functioning of sperm [31, 32].

The Polish patent application PL418751 A1 describes a method for producing health-promoting beer, which is carried out according to traditional stages, starting from malt production, through the wort production stage, the turbulent fermentation stage of the wort, and up to the beer bottling stage. During the wort production stage, a component in the form of angelica (lat. Archangelica officinalis) is added in an amount of 20 - 50 grams per 10 liters of water. Additionally, in this method, the beer is bottled directly after the turbulent fermentation is completed, and the young beer undergoes refermentation and clarification in the vessel, in which it is ultimately ready for consumption. The subject of the application also includes health-promoting beer produced by the above method, containing substances derived from angelica.

The Polish patent application PL432040 A1 discloses a non-alcoholic beer enriched with nutrients, characterized by containing at least one nutrient (folic acid, vitamins, phytosterols, flavonoids, minerals, glucose, fructose, proteins, or amino acids) encapsulated in polymer-coated microcapsules. The application also includes a method for producing the beer, which involves obtaining polymer microcapsules containing at least one nutrient, which are then mixed with the beer.

The Polish patent description No. PL236959 B1 discloses a non-alcoholic functional beverage based on a malt base, non-alcoholic beer, or a water-aromatic base, which contains both an autolysate obtained from yeast slurry waste and fiber derived from crushed brewer's spent grain. The application also includes a method for producing the non-alcoholic functional beverage, which is characterized by the fact that in separate (parallel) technological processes, a suspension of fiber and yeast autolysate is obtained, which are then combined with base ingredients in the form of a malt base, non-alcoholic beer, or water-aromatic base, enriched with flavor and aroma additives, to create a non-alcoholic functional beverage.

The European patent description EP2502635 B1 discloses a preparation composed of two different formulations to be administered separately, one containing arginine, vitamin A, vitamin E, vitamin B6, vitamin B9, vitamin B12, and coenzyme Q10, while the other contains carnitine, arginine, vitamin C, and zinc, for use in the treatment of male infertility.

Currently, the scientific and patent literature does not disclose a dietary supplement based on non-alcoholic beer that, through the content of appropriate microelements and vitamins, supports male fertility and has a positive effect on semen parameters.

The aim of the invention was to develop a novel dietary supplement based on non-alcoholic beer, supporting male fertility, that improves semen parameters, including:
- sperm count,
- sperm motility,
- sperm morphology,
- semen volume.

It is essential that the supplement according to the invention has an easily consumable form of non-alcoholic beer, where the daily dose corresponds to one or two of 325 or 500 ml. The final product, in the form of a dietary supplement, should not differ in taste from other non-alcoholic beers, to make daily supplementation easier for men.

The subject of the invention is a dietary supplement based on non-alcoholic beer that supports male fertility, characterized in that it comprises L-carnitine in the amount of 0.16-0.24% w/w, L-arginine in the amount of 0.02-0.06% w/w, zinc in the amount of 0.002-0.003% w/w, selenium in the amount of 0.00002-0.00004% w/w, vitamin C in the amount of 0.02-0.2% w/w, vitamin E in the amount of 0.01-0.03% w/w, vitamin B6 in the amount of 0.00028-0.002% w/w, vitamin B12 in the amount of 0.0000005-0.000006% w/w, methylfolate in the amount of 0.00008-0.00012% w/w, myo-inositol in the amount of 0.0001-0.0002% w/w, resveratrol in the amount of 0.01-0.04% w/w, choline in the amount of 0.01-0.04% w/w, lycopene in the amount of 0.0002-0.002% w/w, coenzyme Q10 in the amount of 0.001-0.004% w/w

Preferably, when the dietary supplement comprise L-carnitine: in the amount of 0.2% w/w, L-arginine in the amount of 0.04% w/w, zinc in the amount of 0.003% w/w, selenium in the amount of 0.00004% w/w, vitamin C in the amount of 0.1% w/w, vitamin E in the amount of 0.02% w/w, vitamin B6 in the amount of 0.002% w/w, vitamin B12 in the amount of 0.000006% w/w, methylfolate in the amount of 0.00012% w/w, myo-inositol in the amount of 0.0002% w/w, resveratrol in the amount of 0.03% w/w, choline in the amount of 0.03% w/w, lycopene in the amount of 0.0008 w/w, coenzyme Q10 in the amount of 0.003% w/w

The resulting dietary supplement based on non-alcoholic beer according to the invention is characterized by supporting male fertility and positively influencing semen parameters.

Basing the dietary supplement for men on a non-alcoholic beer base may positively influence men's willingness and consistency in using the supplement. It will also allow for reaching out with an educational campaign to those men who avoid doctor visits.

The subject of the invention is further detailed in the examples of its implementation, without limiting its scope.

### Example 1

The composition of the dietary supplement according to the invention (500 ml) has been developed. The composition of individual ingredients is presented in Table 1.

**Table 1**

| Ingredient | Value | NRV |
|---|---|---|
| L-carnitine | 1000 mg | x |
| L-arginine | 200 mg | x |
| Zinc | 15 mg | 10 mg |
| Selenium | 200 µg | 55 ug |
| Vitamin C | 500 mg | 80 mg |
| Vitamin E | 100 mg | 12 mg |
| Vitamin B6 | 10 mg | 1,4 mg |
| Vitamin B12 | 30 µg | 2,5 µg |
| Methylfolate | 600 µg | 600 µg |
| Myo-inositol | 1000 µg | x |
| Resveratrol | 150 mg | x |
| Choline | 150 mg | x |
| Lycopene | 4 mg | x |
| Coenzyme Q10 | 15 mg | x |

| | | |
|---|---|---|
| NRV Nutrient Reference Value | | |

### Example 2

The composition of the dietary supplement according to the invention (500 ml) has been developed. The composition of individual ingredients is presented in Table 2.

**Table 2**

| Ingredient | Value | NRV |
|---|---|---|
| L-carnitine | 800 mg | x |
| L-arginine | 300 mg | x |
| Zinc | 10 mg | 10 mg |
| Selenium | 200 µg | 55 ug |
| Vitamin C | 100 mg | 80 mg |
| Vitamin E | 150 mg | 12 mg |
| Vitamin B6 | 10 mg | 1,4 mg |
| Vitamin B12 | 2,5 µg | 2,5 ug |
| Methylfolate | 400 µg | 600 µg |
| Myo-inositol | 1000 µg | x |
| Resveratrol | 50 mg | x |
| Choline | 200 mg | x |
| Lycopene | 1 mg | x |
| Coenzyme Q10 | 20 mg | x |

| | | |
|---|---|---|
| NRV Nutrient Reference Value | | |

### Example 3

The composition of the dietary supplement according to the invention has been developed. The composition of individual ingredients is presented in Table 3.

**Table 3**

| Ingredient | Value | NRV |
|---|---|---|
| L-carnitine | 1200 mg | x |
| L-arginine | 100 mg | x |
| Zinc | 15 mg | 10 mg |
| Selenium | 100 µg | 55 ug |
| Vitamin C | 1000 mg | 80 mg |
| Vitamin E | 50 mg | 12 mg |
| Vitamin B6 | 1,4 mg | 1,4 mg |
| Vitamin B12 | 30 µg | 2,5 ug |
| Methylfolate | 600 µg | 600 µg |
| Myo-inositol | 500 µg | x |
| Resveratrol | 150 mg | x |
| Choline | 50 mg | x |
| Lycopene | 10 mg | x |
| Coenzyme Q10 | 5 mg | x |

| | | |
|---|---|---|
| NRV Nutrient Reference Value | | |

### Literature:

1. Gou C, Zhou Z, Chen Z, Wang K, Chen C, Chen B, Pan N, He X. Studies on improving semen quality and increasing pregnancy chances through the in vitro addition of L-carnitine and coenzyme Q10 to semen in patients with asthenozoospermia. Basic Clin Androl. 2022 Oct 4;32(1):17. doi: 10.1186/s12610-022-00167-7. PMID: 36192679; PMCID: PMC9531415.
2. Iftikhar A, Akhtar MF, Saleem A, Riaz A, Zehravi M, Rahman MH, Md Ashraf G. Comparative Potential of Zinc Sulfate, L-Carnitine, Lycopene, and Coenzyme Q10 on Cadmium-Induced Male Infertility. Int J Endocrinol. 2022 Jun 30;2022:6266613. doi: 10.1155/2022/6266613. PMID: 35814917; PMCID: PMC9262569
3. Agarwal A, Virk G, Ong C, du Plessis SS. Effect of Oxidative Stress on Male Reproduction World J Mens Health. 2014 Apr;32(1):1- 17. https://doi.org/10.5534/wjmh.2014.32.1.1
4. Balercia G, Mancini A, Paggi F, Tiano L, Pontecorvi A, Boscaro M, Lenzi A, Littarru GP. Coenzyme Q10 and male infertility. J Endocrinol Invest. 2009 Jul;32(7):626-32. doi: 10.1007/BF03346521. Epub 2009 May 21. PMID: 19509475.
5. AlahmarAT, Calogero AE, Singh R, Cannarella R, Sengupta P, Dutta S. Coenzyme Q10, oxidative stress, and male infertility: A review. Clin Exp Reprod Med. 2021 Jun;48(2):97-104. doi: 10.5653/cerm.2020.04175. Epub 2021 May 25. PMID: 34078005; PMCID: PMC8176150.
6. Alahmar AT. The impact of two doses of coenzyme Q10 on semen parameters and antioxidant status in men with idiopathic oligoasthenoteratozoospermia. Clin Exp Reprod Med. 2019 Sep;46(3):112-118. doi: 10.5653/cerm.2019.00136. Epub 2019 Aug 14. PMID: 31408928; PMCID: PMC6736512.
7. Chen JQ, Li YS, Li ZJ, Lu HX, Zhu PQ, Li CM. Dietary I-arginine supplementation improves semen quality and libido of boars under high ambient temperature. Animal. 2018
8. M. Scibona, P. Meschini, S. Capparelli, C. Pecori, P. Rossi, and G. F. M. Fabris, "Arginine and male infertility," Minerva Urologica e Nefrologica, vol. 46, pp. 251-253, 1994. Aug;12(8):1611-1620. doi: 10.1017/S1751731117003147. Epub 2017 Dec 4. PMID: 29198215.
9. Montanino Oliva M, Minutolo E, Lippa A, laconianni P, Vaiarelli A. Effect of Myoinositol and Antioxidants on Sperm Quality in Men with Metabolic Syndrome. Int J Endocrinol. 2016;2016:1674950. doi: 10.1155/2016/1674950. Epub 2016 Sep 26. PMID: 27752262; PMCID: PMC5056296.
10. R. A. Condorelli, S. La Vignera, S. Bellanca, E. Vicari, and A. E. Calogero, "Myoinositol: does it improve sperm mitochondrial function and sperm motility?" Urology, vol. 79, no. 6, pp. 1290-1295, 2012.
11. Moslemi MK, Tavanbakhsh S. Selenium-vitamin E supplementation in infertile men: effects on semen parameters and pregnancy rate. Int J Gen Med. 2011 Jan 23;4:99-104. doi: 10.2147/IJGM.S16275. PMID: 21403799; PMCID: PMC3048346.
12. Scott R, MacPherson A, Yates RW, Hussain B, Dixon J. The effect of oral selenium supplementation on human sperm motility. BrJ Urol. 1998 Jul;82(1):76-80. doi: 10.1046/j.1464-410x.1998.00683.x. PMID: 9698665.
13. Lippman SM, Klein EA, Goodman PJ, Lucia MS, Thompson IM, Ford LG, Parnes HL, Minasian LM, Gaziano JM, Hartline JA, Parsons JK, Bearden JD 3rd, Crawford ED, Goodman GE, Claudio J, Winquist E, Cook ED, Karp DD, Walther P, Lieber MM, Kristal AR, Darke AK, Arnold KB, Ganz PA, Santella RM, Albanes D, Taylor PR, Probstfield JL, Jagpal TJ, Crowley JJ, Meyskens FL Jr, Baker LH, Coltman CA Jr. Effect of selenium and vitamin E on risk of prostate cancer and other cancers: the Selenium and Vitamin E Cancer Prevention Trial (SELECT). JAMA. 2009 Jan 7;301(1):39-51. doi: 10.1001/jama.2008.864. Epub 2008 Dec 9. PMID: 19066370; PMCID: PMC3682779.
14. Reid ME, Duffield-Lillico AJ, Slate E, Natarajan N, Turnbull B, Jacobs E, Combs GF Jr, Alberts DS, Clark LC, Marshall JR. The nutritional prevention of cancer: 400 mcg per day selenium treatment. Nutr Cancer. 2008;60(2):155-63. doi: 10.1080/01635580701684856. PMID: 18444146.
15. Allouche-Fitoussi D, Breitbart H. The Role of Zinc in Male Fertility. Int J Mol Sci. 2020 Oct 21;21(20):7796. doi: 10.3390/ijms21207796. PMID: 33096823; PMCID: PMC7589359.
16. Colagar A.H., Marzony E.T., Chaichi M.J. Zinc levels in seminal plasma are associated with sperm quality in fertile and infertile men. Nutr. Res. 2009;29:82-88. doi: 10.1016/j.nutres.2008.11.007.
17. Liu D.Y., Sie B.S., Liu M.L., Agresta F., Baker H.W. Relationship between seminal plasma zinc concentration and spermatozoa-zona pellucida binding and the ZP-induced acrosome reaction in subfertile men. Asian J. Androl. 2009;11:499-507. doi: 10.1038/aja.2009.23.
18. Hunt C.D., Johnson P.E., Herbel J., Mullen L.K. Effects of dietary zinc depletion on seminal volume and zinc loss, serum testosterone concentrations, and sperm morphology in young men. Am. J. Clin. Nutr. 1992;56:148-157. doi: 10.1093/ajcn/56.1.148.
19. Zdrojewicz Z, Wiśniewska A Rola cynku w seksualności m 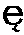 żczyzn; Adv Clin Exp Med 2005, 14, 6, 1295-1300)
20. Sabetian S, Jahromi BN, Vakili S, Forouhari S, Alipour S. The Effect of Oral Vitamin E on Semen Parameters and IVF Outcome: A Double-Blinded Randomized Placebo-Controlled Clinical Trial. Biomed Res Int. 2021 Oct 11;2021:5588275. doi: 10.1155/2021/5588275. PMID: 34671676; PMCID: PMC8523239.
21. Zhou X, Shi H, Zhu S, Wang H, Sun S. Effects of vitamin E and vitamin C on male infertility: a meta-analysis. Int Urol Nephrol. 2022 Aug;54(8):1793-1805. doi: 10.1007/s11255-022-03237-x. Epub 2022 May 23. PMID: 35604582.
22. Clagett-Dame M, Knutson D. Vitamin A in reproduction and development. Nutrients. 2011 Apr;3(4):385-428. doi: 10.3390/nu3040385. Epub 2011 Mar 29. PMID: 22254103; PMCID: PMC3257687.
23. Hogarth CA, Griswold MD. The key role of vitamin A in spermatogenesis. J Clin Invest. 2010 Apr;120(4):956-62. doi: 10.1172/JCI41303. Epub 2010 Apr 1. PMID: 20364093; PMCID: PMC2846058.
24. Durairajanayagam D, Agarwal A, Ong C, Prashast P. Lycopene and male infertility. Asian J Androl. 2014 May-Jun;16(3):420-5. doi: 10.4103/1008-682X.126384. PMID: 24675655; PMCID: PMC4023371.
25. Chen P, Zhang W, Wang X, Zhao K, Negi DS, Zhuo L, Qi M, Wang X, Zhang X. Lycopene and Risk of Prostate Cancer: A Systematic Review and Meta-Analysis. Medicine (Baltimore). 2015 Aug;94(33):e1260. doi: 10.1097/MD.0000000000001260. PMID: 26287411; PMCID: PMC4616444.
26. Lu Y, Edwards A, Chen Z, Tseng T-S, Li M, Gonzalez GV and Zhang K (2021) Insufficient Lycopene Intake Is Associated With High Risk of Prostate Cancer: A Cross-Sectional Study From the National Health and Nutrition Examination Survey (2003-2010). Front. Public Health 9:792572. doi: 10.3389/fpubh.2021.792572
27. Banihani SA. A Systematic Review Evaluating the Effect of Vitamin B6 on Semen Quality. Urol J. 2017 Dec 30;15(1):1-5. doi: 10.22037/uj.v0i0.3808. Epub ahead of print. PMID: 29290084.
28. Mathieu d'Argent E, Ravel C, Rousseau A, Morcel K, Massin N, Sussfeld J, Simon T, Antoine JM, Mandelbaume J, Daraï E, Kolanska K. High-Dose Supplementation of Folic Acid in Infertile Men Improves IVF-ICSI Outcomes: A Randomized Controlled Trial (FOLFIV Trial). J Clin Med. 2021 Apr 26;10(9):1876. doi: 10.3390/jcm10091876. PMID: 33925981; PMCID: PMC8123699.
29. Schisterman EF, Sjaarda LA, Clemons T, Carrell DT, Perkins NJ, Johnstone E, Lamb D, Chaney K, Van Voorhis BJ, Ryan G, Summers K, Hotaling J, Robins J, Mills JL, Mendola P, Chen Z, DeVilbiss EA, Peterson CM, Mumford SL. Effect of Folic Acid and Zinc Supplementation in Men on Semen Quality and Live Birth Among Couples Undergoing Infertility Treatment: A Randomized Clinical Trial. JAMA. 2020 Jan 7;323(1):35-48. doi: 10.1001/jama.2019.18714. Erratum in: JAMA. 2020 Mar 24;323(12):1194. PMID: 31910279; PMCID: PMC6990807.
30. Wong WY, Merkus HM, Thomas CM, Menkveld R, Zielhuis GA, Steegers-Theunissen RP. Effects of folic acid and zinc sulfate on male factor subfertility: a double-blind, randomized, placebo-controlled trial. Fertil Steril. 2002 Mar;77(3):491-8. doi: 10.1016/s0015-0282(01)03229-0. PMID: 11872201.
31. Hinkovska VT, Dimitrov GP, Koumanov KS. Phospholipid composition and phospholipid asymmetry of ram spermatozoa plasma membranes. Int J Biochem. 1986;18(12):1115-21. doi: 10.1016/0020-711x(86)90085-6. PMID: 3817272.
32. Shan S, Xu F, Hirschfeld M, Brenig B. Sperm Lipid Markers of Male Fertility in Mammals. Int J Mol Sci. 2021 Aug 16;22(16):8767. doi: 10.3390/ijms22168767. PMID: 34445473; PMCID: PMC8395862.
33. Skoracka K, Eder P, Lykowska-Szuber L, Dobrowolska A, Krela-Kaźmierczak I. Diet and Nutritional Factors in Male (In)fertility-Underestimated Factors. J Clin Med. 2020 May 9;9(5):1400. doi: 10.3390/jcm9051400. PMID: 32397485; PMCID: PMC7291266

## Claims

1. A dietary supplement based on non-alcoholic beer supporting male fertility, **characterized in that** it comprises L-carnitine in an amount of 0.16-0.24% w/w, L-arginine in an amount of 0.02-0.06% w/w, zinc in an amount of 0.002-0.003% w/w, selenium in an amount of 0.00002-0.00004% w/w, vitamin C in an amount of 0.02-0.2% w/w, vitamin E in an amount of 0.01-0.03% w/w, vitamin B6 in an amount of 0.00028-0.002% w/w, vitamin B12 in an amount of 0.0000005-0.000006% w/w, methylfolate in an amount of 0.00008-0.00012% w/w, myo-inositol in an amount of 0.0001-0.0002% w/w, resveratrol in an amount of 0.01-0.04% w/w, choline in an amount of 0.01-0.04% w/w, lycopene in an amount of 0.0002-0.002% w/w, coenzyme Q10 in an amount of 0.001-0.004% w/w.

2. A dietary supplement according to claim 1, **characterized in that** it comprises L-carnitine in an amount of 0.2% w/w, L-arginine in an amount of 0.04% w/w, zinc in an amount of 0.003% w/w, selenium in an amount of 0.00004% w/w, vitamin C in an amount of 0.1% w/w, vitamin E in an amount of 0.02% w/w, vitamin B6 in an amount of 0.002% w/w, vitamin B12 in an amount of 0.000006% w/w, methylfolate in an amount of 0.00012% w/w, myo-inositol in an amount of 0.0002% w/w, resveratrol in an amount of 0.03% w/w, choline in an amount of 0.03% w/w, lycopene in an amount of 0.0008% w/w, coenzyme Q10 in an amount of 0.003% w/w.
